# EUROPEAN PATENT APPLICATION

(11) **EP 4 578 371 A1**
(43) Date of publication of application: **02.07.2025**
(21) Application number: 24189203.3
(22) Date of filing: 17.07.2024
(51) Int. Cl.: A61B 1/00, A61B 1/012, A61B 1/015, A61B 1/04, A61B 1/06

(54) **MEDICAL DEVICE WITH INTEGRATED OUTER SHEATH**

(30) Priority: 25.12.2023 CN 202311800316
(71) Applicant: Zhuhai Pusen Medical Technology Co., Ltd., Zhuhai, Guangdong 519085 (CN)
(72) Inventor: HUANG, Honghui, Zhuhai, 519085 (CN); LI, Baohua, Zhuhai, 519085 (CN)
(74) Representative: Zaboliene, Reda

(57) **Abstract**

Disclosed is a medical device with an integrated outer sheath, includes a device body and a sheath, the device body includes an inserting portion and a holding portion, a tip end of the inserting portion is provided with a distal tip, a penetrating first through hole and a second through hole are arranged on the distal tip, a first tube is connected to the first through hole; both ends of the sheath are respectively connected and sealed with the inserting portion and the holding portion through a connecting structure, the sheath is detachably and rotatably sleeved outside the inserting portion to form an overtube of the inserting portion, the sheath is sleeved outside the first tube, a gap between the sheath and the first tube is a space used by the sheath to transport objects. Improves the performance of the device, while the outer diameter of the device remains unchanged.

## Description

### TECHNICAL FIELD

The present disclosure herein relates to the field of medical device, and in particular to a medical device with an integrated outer sheath.

### BACKGROUND

Medical devices such as endoscopes or sheaths are devices that enter the body through the natural channels of the human body to perform detection or treatment. Various types of endoscopes and sheaths can be used for surgical and exploratory operations, most of which involve the inlet and outlet of the device and the inflow and discharge of fluid, two channels are usually required in this type of medical device, one channel for the inlet and outlet of the device, and another channel for the inlet and outlet of the liquid. Based on the background of medical devices being used in the body, the outer diameter of the inserting portion of the device is required to be as small as possible and the internal working channel is as large as possible. When multiple channels are provided, the size of the channels will be limited, and the size of the distal tip will also be increased and the size of the camera instrument will be reduced, which will affect the performance and effect of the device.

Moreover, in many cases, medical devices such as endoscopes and sheaths are used together. The gap between the sheath and the endoscope is small, and the inlet and outlet rate of liquid is low, making the endoscope easily obscured by body fluids, resulting in blurry and unclear images, poor stone removal effect, and slow operation efficiency. There is a need for a medical device that can expand the channel size of the sheath without increasing the size of the distal tip of the endoscope.

### SUMMARY

In order to overcome at least one of the aforementioned technical problems of the prior art, the present disclosure provides a medical device with an integrated outer sheath that uses an outer sheath as the overtube of the device to increase the number of channels inside the device without increasing the outer diameter of the inserting portion.

Disclosed are a medical device with an integrated outer sheath, which includes a device body and a sheath, the device body includes an inserting portion and a holding portion connected front and back, a first outlet and a second outlet are arranged on the holding portion, a tip end of the inserting portion is provided with a distal tip, a penetrating first through hole and a second through hole are arranged on the distal tip, a first tube is connected to the first through hole, and a terminal end of the first tube extends into the holding portion and communicates with the first outlet; both ends of the sheath are connected and sealed with the inserting portion respectively through a connecting structure, so that the sheath is detachably and rotatably sleeved outside the inserting portion to form an overtube of the inserting portion; a distal end of the sheath is communicated with the second through hole of the distal tip, a terminal end of the sheath is communicated with the second outlet of the holding portion, the sheath is sleeved outside the first tube, and a gap between the sheath and the first tube is a space used by the sheath to transport objects.

The aforementioned medical device has at least the following beneficial effects: the sheath is used as the overtube of the device, and the device body and the sheath are detachably connected. The two can be assembled and placed into the body at the same time, or the sheath can be placed into the body separately, or the device body can be removed out separately, eliminating the thickness of the original overtube of the device, allowing the diameter of the device body to be increased, increasing the diameter of the through hole of the device body, thereby improving the performance of the device. By using the gap between the first tube and the sheath as the second channel, the number of channels inside the device can be increased, and the internal space of the second channel is large, which increases the flow rate of the fluid being transported and improves the efficiency of transportation.

In some embodiments of the aforementioned medical device, the connecting structure includes a snap-fit connection, an interference fit connection, a threaded connection, a magnetic connection, and combination of the above connection methods. The sheath can be sealed and connected to the holding portion, ensuring that the connection between the sheath and the holding portion is fast and firm.

In some embodiments of the aforementioned medical device, at least one sealing member is arranged between an inner wall of the sheath and an outer wall of the inserting portion, the sealing member is positioned on one side of the connecting structure away from an end portion of the inserting portion, and the sealing member is fixed on the inner wall of the sheath and/or the outer wall of the inserting portion. The sealing member can enhance the sealing effect between the end portion of the sheath and the inserting portion, ensuring that the fluid transported in the sheath will not leak from the connection between the both ends of the sheath.

In some embodiments of the aforementioned medical device, an inner diameter of the distal end of the sheath is smaller than an inner diameter of other portions of the sheath. Reducing the diameter of the distal end of the sheath can facilitate the placement of the sheath and the device body into the body, and can also enhance the sealing effect between the distal end of the sheath and the inserting portion.

In some embodiments of the aforementioned medical device, the sheath is an elastically deformable flexible tube. The flexible sheath possesses the ability to deform. When the sheath is inserted and withdrawn independently, the wall of the sheath is squeezed inward by the pressure inside the body. The flexible wall can deform and collapse inward, thereby reducing the diameter of the sheath and increasing the distance between its outer wall and the channel within the body. This reduction in diameter decreases the risk of the sheath being in close contact with a narrow lumen and becoming immobile.

In some embodiments of the aforementioned medical device, both the inner wall and the outer wall of the sheath are provided with a hydrophilic layer. The hydrophilic layer can become lubricated upon contact with liquid, which facilitates the insertion of the sheath into the human body and reduces the difficulty of operating the sheath. The inner wall of the sheath becomes lubricated after being moistened, which can reduce the friction between the sheath and the inserting portion. The resistance between the sheath and the inserting portion is smaller, making it easier for the sheath or the inserting portion to rotate independently without affecting the movement of the other.

In some embodiments of the aforementioned medical device, the outer wall of the sheath is provided with raised patterns. The raised patterns prevent the sheath from rotating. When the inserting portion rotates, it ensures that the sheath will not rotate together, preventing medical accidents caused by sheath rotation and further improving the safety of the device.

In some embodiments of the aforementioned medical device, the inserting portion further includes a protective tube, at least one end of the protective tube is connected to the distal tip or the holding portion, the protective tube is sleeved outside the first tube, the sheath is sleeved outside the protective tube, and an inside and outside of the protective tube are interconnected. The diameter of the protective tube is larger than that of the first tube. At the bending part, the bending radius of the protective tube is larger than that of the first tube, which can prevent the first tube from breaking due to an excessive bending angle, improve the durability of the device, and reduce the probability of accidents. The internal and external communication of the protective tube can transport the fluid in the protective tube to the outside of the protective tube, ensuring the transport space between the sheath and the first tube.

In some embodiments of the aforementioned medical device, a plurality of leakage holes are arranged on the protective tube. The leakage hole can pass fluid and increase the flow rate of fluid on both the inside and outside of the protection tube, making the discharge or extraction of fluid more efficient.

In some embodiments of the aforementioned medical device, an anti-slip structure is arranged on the outer wall of a proximal end of the sheath. The sheath usually comes into contact with liquid when used. After being wet, the sheath becomes slippery and difficult to hold. The anti-slip structure is provided to facilitate the holding of the sheath and the connection or separation of the sheath and the holding portion, making it less likely to slip.

### BRIEF DESCRIPTION OF THE DRAWINGS

In order to explain the technical solutions in the embodiments of the present disclosure more clearly, the accompanying drawings needed to be used in the description of the embodiments will be briefly described below. Obviously, the described accompanying drawings are only some of the embodiments of the present disclosure, not all embodiments. A person skilled in the art can also obtain other design solutions and drawings based on these drawings without exerting creative efforts:
FIG. 1 is a structure schematic diagram according to the first embodiment of the present disclosure;
FIG. 2 is an exploded schematic diagram according to the first embodiment of the present disclosure;
FIG. 3 is a front view of a distal tip according to the first embodiment of the present disclosure;
FIG. 4 is a partial cross-sectional view according to the first embodiment of the present disclosure;
FIG. 5 is a partial cross-sectional view according to the second embodiment of the present disclosure;
FIG. 6 is a cross-sectional view of a connection between a sheath and a distal tip according to the third embodiment of the present disclosure;
FIG. 7 is a partial cross-sectional view according to the fourth embodiment of the present disclosure;
FIG. 8 is an exploded schematic diagram according to the fifth embodiment of the present disclosure;
FIG. 9 is a partial cross-sectional view according to the fifth embodiment of the present disclosure; and
FIG.10 is a partial cross-sectional view according to the sixth embodiment of the present disclosure.

### DETAILED DESCRIPTION OF THE EMBODIMENTS

In the description of the present disclosure, it should be noted that the terms such as "center," "upper," "lower," "left," "right," "vertical," "horizontal," "inner," "outer," and other indications of orientation or positional relationships are based on the orientation or positional relationships shown in the drawings. They are used solely for the purpose of facilitating the description of the present disclosure and simplifying it, and are not intended to indicate or imply that the instruments or elements referred to must have a specific orientation, be constructed in a specific orientation, or be operated in a specific way. Therefore, they should not be construed as limitations to the present disclosure. Furthermore, the terms "first", "second" and "third" are used for descriptive purposes only and are not to be construed as indicating or implying relative importance.

It should be noted that, unless otherwise specified or limited, the terms "installation," "connected," and "connection" should be understood in a broad sense. For example, they can refer to a fixed connection, a detachable connection, or an integral connection; they can be a mechanical connection, or an electrical connection; they can be a direct connection, or an indirect connection through an intermediate medium; and they can also refer to the internal communication between two elements. For those of ordinary skill in the art, the specific meanings of the above terms in the present disclosure can be understood on a case-by-case basis.

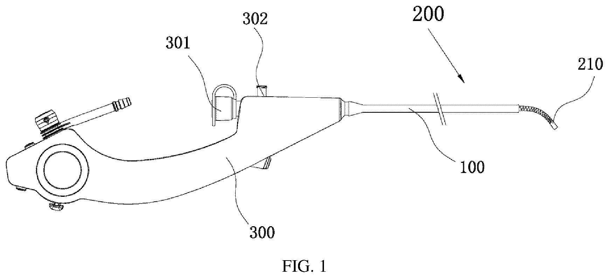

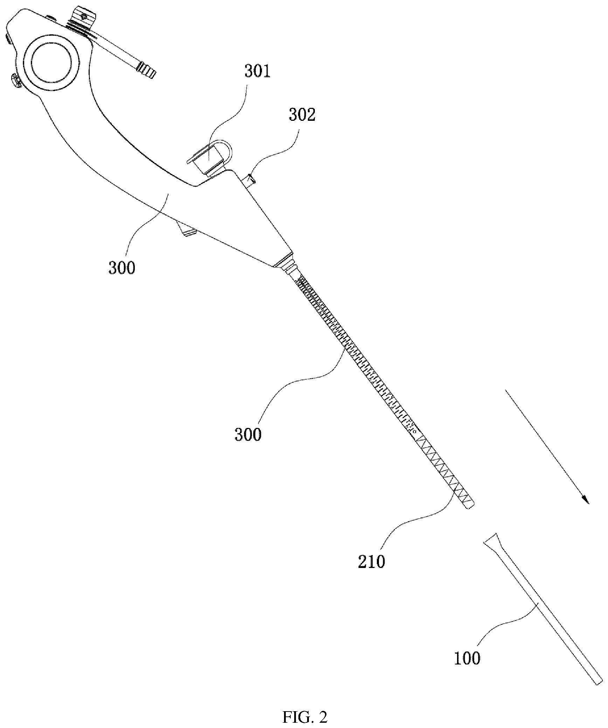

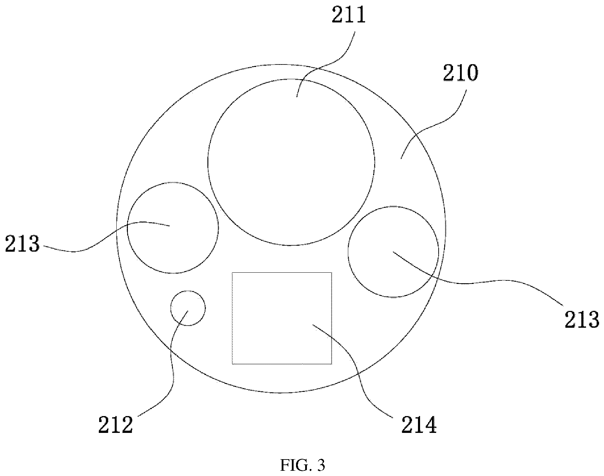

Referring to FIGS. 1 to 5, a medical device with an integrated outer sheath is disclosed, including a device body and a sheath 100. The device body refers to medical devices that need to enter the human body channels, such as endoscopes, biopsy needles, suction tubes, etc. The device body can be a flexible device with controllable bending or a rigid device that cannot be bent.

The device body includes an inserting portion 200 and a holding portion 300 connected front and back, a first outlet 301 and a second outlet 302 are arranged on the holding portion 300, a tip end of the inserting portion 200 is provided with a distal tip 210, a penetrating first through hole 211 and a second through hole 212 are arranged on the distal tip 210, a first tube 220 is connected to the first through hole 211, a terminal end of the first tube 220 extends into the holding portion 300 and communicates with the first outlet 301, and the first tube 220 constitutes a first channel. When the device body is an endoscope, an illuminating instrument 213 and a camera instrument 214 are also fixed in the distal tip 210. When the device body is a biopsy needle, the distal end of the distal tip 210 is configured as a biopsy needle tip. When the device body is a suction tube, the distal end of the distal tip 210 is provided with a suction through hole. In other embodiments, the structure of the distal tip 210 has corresponding changes according to different types of device bodies.

Both ends of the sheath 100 are connected and sealed with the inserting portion 200 respectively through a connecting structure, so that the sheath 100 is detachably and rotatably sleeved outside the inserting portion 200, and the sheath 100 forms an overtube of the inserting portion 200. During use, the sheath 100 can be placed into the human body channel first, and then the inserting portion 200 of the device body is placed into the body through the sheath 100. When the inserting portion 200 moves into place, the distal tip 210 extends out of the sheath 100, and the distal end and the proximal end of the sheath 100 are respectively connected to the device body through the connecting structure, and seal the both ends of the sheath 100. The sheath 100 can rotate relative to the inserting portion 200 and the holding portion 300 along the axis. When the inserting portion 200 is controlled to rotate and adjust the direction of the distal tip 210, the sheath 100 can remain relatively stationary or rotate slightly. The connecting structure allows for the relative rotation between the two components, preventing the rotation of the sheath 100 from driving the rotation of the human body channel, avoiding different degrees of medical accidents such as twisting or tearing of the internal cavity, and making the use of the instrument safer and reducing the risk of use.

The sheath 100 is sleeved outside the inserting portion 200. At this time, both ends of the sheath 100 are closed, a distal end of the sheath 100 is communicated with the second through hole 212 of the distal tip 210, a terminal end of the sheath 100 is communicated with the second outlet 302 of the holding portion 300, and the sheath 100 constitutes a second channel. The sheath 100 is sleeved outside the first tube 220, and a gap between the sheath 100 and the first tube 220 is a space used by the sheath 100 to transport objects. The aforementioned "communication" refers to the connectivity between channels. In addition to being directly connected, the sheath 100 can also be indirectly connected to the second through hole 212 or the second outlet 302 through other components, allowing for the communication and ventilation of the channels.

The first channel extends from the first through hole 211 of the distal tip 210 along the first tube 220 to the first outlet 301 of the holding portion 300. The second channel extends from the second through hole 212 of the distal tip 210 along the sheath 100 to the second outlet 302 of the holding portion 300. The second channel utilizes the gap between the inner wall of the sheath 100 and the outer wall of the first tube 220 in the inserting portion 200.

In some embodiments, a connecting tube 310 is arranged in the holding portion 300, the distal end of the connecting tube 310 is connected to the sheath 100, and the terminal end of the connecting tube 310 is connected to the second outlet 302. The connection between the sheath 100 and the second outlet 302 of the holding portion 300 is achieved via the connecting tube 310.

The connecting structure is arranged on the inner walls of both ends of the sheath 100. The connecting structure includes a snap-fit connection, an interference fit connection, a threaded connection, a magnetic connection, and combination of the above connection methods. The connecting structure includes a front connecting structure connecting the sheath 100 and the tip end of the inserting portion 200, and a rear connecting structure connecting the sheath 100 and the inserting portion 200 or the holding portion 300. Both the front connecting structure and the rear connecting structure include detachably connected limiting component and connecting component. The limiting component and connecting component are selectively arranged at the end portion of the sheath 100, with the other being arranged on the corresponding end of the inserting portion 200 or the holding portion 300. Depending on the connecting structure, the limiting component and the connecting component can be different components such as bumps and grooves, magnetic objects that attract each other, two contact surfaces that are in close contact with each other, or threads that fit into each other.

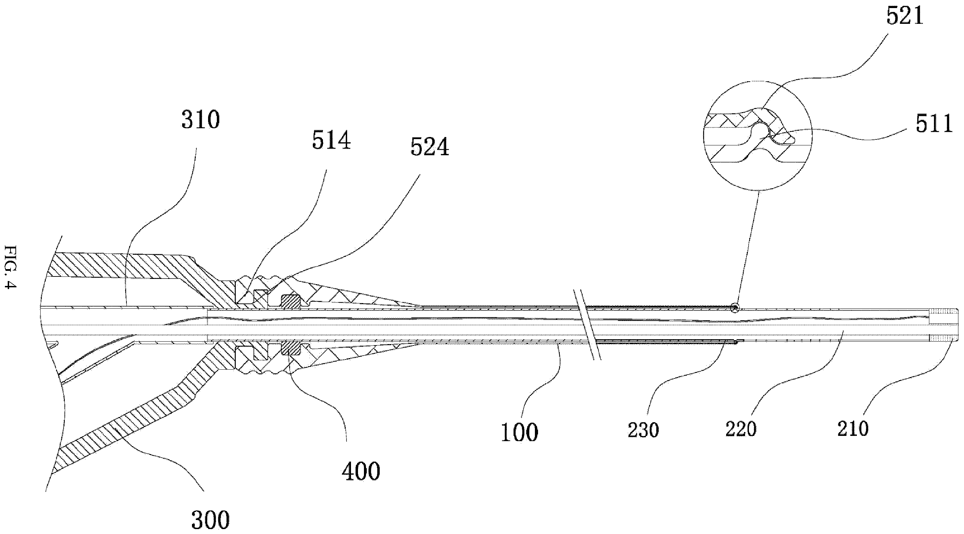

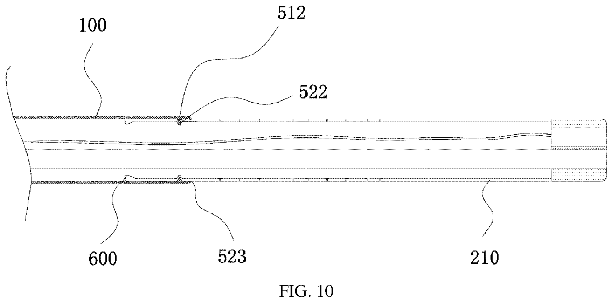

Referring to the embodiments of FIGS. 4 to 9, the front connecting structure in the above embodiment includes a protrusion 511 arranged on the inserting portion 200 and a groove 521 arranged on the sheath 100. The protrusion 511 is positioned on the outer wall of the distal end section of the inserting portion 200, and the groove 521 is formed by an outward protrusion from the wall of the distal end of the sheath 100. When the inserting portion 200 passes through the sheath 100 and moves to the foremost end of the sheath 100, the protrusion 511 pushes into the groove 521, and the protrusion 511 is blocked by the foremost end side wall of the groove 521, so that the distal end of the sheath 100 can be firmly clamped to achieve sealing and fixation of the distal end of the sheath 100 and the inserting portion 200. In the embodiment of FIG. 10, the front connecting structure includes a groove 522 arranged on the inserting portion 200 and a protrusion 512 arranged on the sheath 100. The groove 522 is positioned on the outer wall of the distal end of the inserting portion 200, and the protrusion 512 is positioned on the inner wall of the distal end of the sheath 100.

In addition to the aforementioned snap connection, in other embodiments not shown, the sheath 100 and the inserting portion 200 can also be connected through threads, or through a plurality of annular grooves and limiting blocks embedded in each other, or by providing a magnetic coating so that the two portions are magnetically connected, or by reducing the diameter of the distal end of the sheath 100 so that the sheath 100 and the inserting portion 200 are closely connected, or by combining a snap-fit connection with an interference fit connection to connect the sheath 100 to the inserting portion 200. The diameter of the distal end of the sheath 100 is reduced, at the same time, a protrusion is arranged on the inserting portion 200 to connect the sheath 100 and the inserting portion 200. The connecting structure between the sheath 100 and the distal end of the inserting portion 200 only needs to be detachable, rotatable, and sealed.

Furthermore, in the embodiment of FIG. 10, the sheath 100 is also provided with a plurality of front connecting structures. The distal end portion of the sheath 100 is bent inward, and a recessed second groove 523 is also arranged on the outer wall of the distal tip 210. The distal end portion of the sheath 100 is snapped into the second groove 523 to further fix the position of the sheath 100. A plurality of front connecting structures are arranged to ensure that the sheath 100 can be firmly snapped outside the inserting portion 200, making it difficult for the sheath 100 to detach from the inserting portion 200, thereby realizing the sealing and connection of the distal end of the sheath 100.

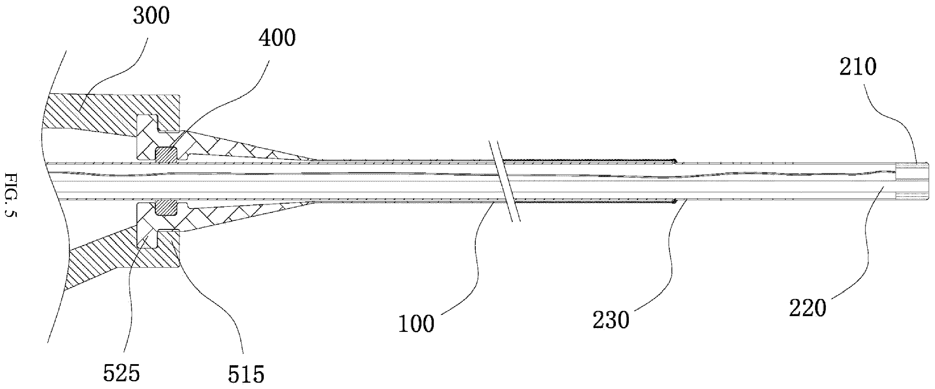

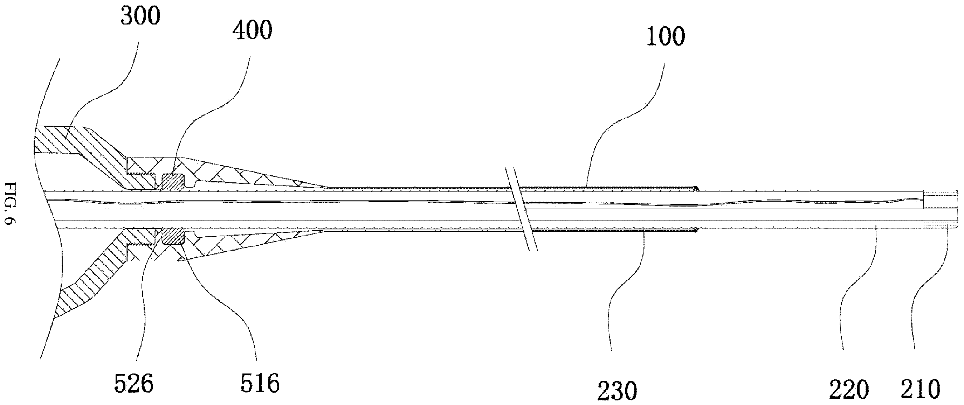

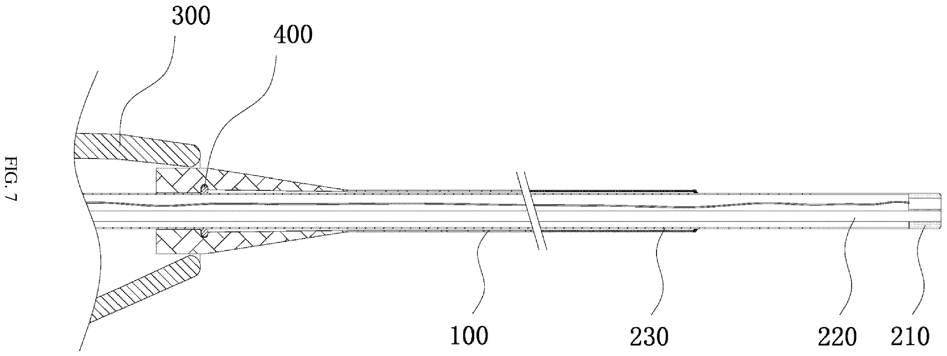

Referring to the embodiment of FIG. 4, the rear connecting structure of the above embodiment includes a groove 524 arranged on the outer wall of the holding portion 300, and a first clamping block 514 arranged on the inner wall of the proximal end portion of the sheath 100. The first clamping block 514 is pushed into the groove 524 and connected to each other to achieve sealing and fixation of the proximal end of the sheath 100 and the holding portion 300. In the embodiment of FIG. 5, the rear connecting structure includes a limiting hook 515 arranged at the distal end of the holding portion 300, and a second clamping block 525 arranged on the outer wall of the proximal end portion of the sheath 100. The second clamping block 525 of the sheath 100 is snapped into the limiting hook 515 for connection and fixing. In addition to the aforementioned snap-fit structure, in the embodiment of FIG. 6, the inner wall of the sheath 100 and the outer wall of the holding portion 300 are provided with threads that match each other, and the two are connected through threads. In the embodiment of FIG. 7, the terminal end of the sheath 100 and the terminal end of the inserting portion 200 are snap-connected through the limiting ring 516 and the limiting groove 526. In other embodiments not shown, the proximal end of the sheath 100 can also be connected to the holding portion 300 through a magnetic snap-fit structure in which a magnetic ring and a slot cooperate with each other. The rear connecting structure is not limited, as long as the rear connecting structure between the sheath 100 and the proximal end of the inserting portion 200 or the holding portion 300 meets the detachable and rotatable sealing connection.

In some embodiments, at least one sealing member 400 is arranged between an inner wall of the sheath 100 and an outer wall of the inserting portion 200, the sealing member 400 is positioned on one side of the connecting structure away from an end portion of the inserting portion 200, and the sealing member 400 is fixed on the inner wall of the sheath 100 and/or the outer wall of the inserting portion 200. The sealing member 400 is arranged at the proximal end of the front connecting structure or the distal end of the rear connecting structure. The sealing member 400 is positioned on one side of the connecting structure and away from the end portion of the inserting portion 200, which can prevent the fluid inside the inserting tube 200 from leaking out through the connecting structure and avoid a decrease in the inflow or outflow speed of the fluid. Referring to the embodiments in FIGS. 4 to 6, the sealing member 400 can be an annular sealing ring. Alternatively, the sealing member 400 can also be arranged as an elastic sheet with an opening in the center, or an elastic film with an opening, or a plurality of elastic pieces arranged axially and spaced apart along the sheath 100. The structure of the sealing member 400 is not limited, as long as it can seal and prevent leakage and allow the first tube 220 to pass through.

In some embodiments, the caliber of the distal end of the sheath 100 is smaller than the inner diameter of other portions of the sheath 100. Reducing the diameter of the distal end of the sheath 100 can facilitate the placement of the sheath 100 into the body, and can also enhance the sealing effect between the distal end of the sheath 100 and the inserting portion 200, making the connection between the distal end of the sheath 100 and the inserting portion 200 stronger.

Furthermore, the sheath 100 is an elastically deformable flexible tube. The flexible sheath 100 possesses the ability to deform. When the sheath 100 is inserted and withdrawn independently, the wall of the sheath 100 is squeezed inward by the pressure inside the body. The flexible wall can deform and collapse inward, thereby reducing the diameter of the sheath 100 and increasing the distance between its outer wall and the channel within the body. This reduction in diameter decreases the risk of the sheath 100 being in close contact with a narrow lumen and becoming immobile. Moreover, the flexible sheath 100 can be bent easily and can be used in conjunction with flexible medical devices.

In some embodiments, the outer wall of the sheath 100 is provided with a hydrophilic layer, and the inner wall of the sheath 100 is also provided with a hydrophilic layer. Specifically, the entire sheath 200 is made of hydrophilic lubricating material, or the outer and inner walls of the sheath 200 are coated with a hydrophilic lubricating coating, or the inner and outer walls of the sheath 100 are made of hydrophilic lubricating materials. The hydrophilic layer on the outer wall makes the outer wall of the sheath 100 relatively lubricated, which reduces the friction between the outer wall of the sheath 100 and the human body channel wall, making it easier for the sheath 100 to be inserted into the body cavity, and also facilitates the rotation of the sheath 100. The hydrophilic coating on the inner wall reduces the resistance between the sheath 100 and the inserting portion 200. The sheath 100 and the inserting portion 200 can rotate relative to each other along the axis. When the inserting portion 200 rotates, the sheath 100 can remain relatively stationary or rotate slightly. When the sheath 100 rotates, the inserting portion 200 can also remain relatively stationary or rotate slightly. The hydrophilic coating on the inner wall of the sheath 100 makes the relative rotation smoother, making the device more convenient to operate and use, and can also avoid accidents in which the sheath 100 is torsion and causes torsion and tearing of the body cavity, thereby reducing the risk of use.

In an embodiment not shown, the outer wall of the sheath 100 is also provided with raised patterns. Preferably, the patterns are stripe patterns arranged along the axial direction of the sheath 100, which can be in different shapes such as straight lines, threads, arcs, etc. The raised patterns prevent the sheath 100 from rotating. When the inserting portion 200 rotates, it ensures that the sheath 100 will not rotate together, preventing medical accidents caused by sheath 100 rotation and further improving the safety of the device.

Furthermore, an anti-slip structure 130 is arranged on the outer wall of a proximal end of the sheath 100. The sheath 100 usually comes into contact with liquid when used. After being wet, the sheath 100 becomes slippery and difficult to hold. The anti-slip structure is provided to facilitate the holding of the sheath 100 and the connection or separation of the sheath 100 and the holding portion 300, making it less likely to slip. The anti-slip structure can be a raised anti-slip texture, or be covered with an anti-slip coating, or be provided with a holding handle, or, with reference to Embodiment 1 of FIG. 4, the outer wall of the terminal end of the sheath is configured to conform to the shape of the fingers when holding it. It has an ergonomic arc shape, and the anti-slip structure scheme is not limited, as long as it has an anti-slip effect.

In some embodiments, the inserting portion 200 further includes a protective tube 230, at least one end of the protective tube 230 is connected to the distal tip 210 or the holding portion 300, the protective tube 230 is sleeved outside the first tube 220, the sheath 100 is sleeved outside the protective tube 230, and an inside and outside of the protective tube 230 are interconnected. The diameter of the protective tube 230 is larger than that of the first tube 220, which can enhance the strength of the inserting portion 200 and make it easier to insert the inserting portion 200 when it is inserted into the body. When bending, the bending radius of the protective tube 230 is larger than that of the first tube 220, which can prevent the first tube 220 from breaking due to an excessive bending angle, improve the durability of the device, and reduce the probability of accidents. The internal and external communication of the protective tube 230 can transport the fluid in the protective tube 230 to the outside of the protective tube 230, ensuring the transport space between the sheath 100 and the first tube 220. Furthermore, in order to increase the flow rate of fluid on both sides of the protective tube 230 and make the discharge or extraction of fluid more efficient, a plurality of leakage holes are also arranged on the protective tube 230.

Referring to the embodiments of FIGS. 1 to 7, a protective tube 230 is also connected to the inserting portion 200. The distal end of the protective tube 230 is connected and fixed to the distal tip 210, and the proximal end of the protective tube 230 is fixed in the holding portion 300. The connecting tube 300 of the holding portion 300 is connected to the terminal end of the protective tube 230. The protective tube 230 is also provided with an array of slits for bending, and the slit arrays can also be used as leakage holes for fluid to pass through. In other embodiments not shown, the distal end of the protective tube 230 may be connected to the distal tip 210 and the proximal end may not be fixed for transporting fluid, or the proximal end of the protective tube 230 may be fixed to the holding portion 300 or the inserting portion 200, while the distal end is not fixed for transporting fluid. The fixation of the protective tube 230 and the exchange of internal and external fluids are ensured.

In the first embodiment shown in FIGS. 1 to 4, in addition to the first through hole 211 and the second through hole 212, the distal tip 210 of the device body is also provided with a lighting hole 213 for installing a lighting instrument and a camera hole 214 for installing a camera instrument. The connecting cables of the camera instrument and the lighting instrument pass through the distal tip 210 and extend into the holding portion 300 together with the first tube 220. A protective tube 230 is fixed at the proximal end of the distal tip 210. The protective tube 230 is sleeved outside the first tube 220 and the connecting cable. The terminal end of the protective tube 230 is fixed in the holding portion 300. A connecting tube 310 is arranged in the holding portion, and the terminal end of the protective tube 230 is connected to the connecting tube 310. Both ends of the sheath 100 are connected to the protective tube 230 and the holding portion 300 via a snap-fit structure. After connection, the fluid enters from the second outlet 302 of the holding portion 300 and flows into the protective tube 230 of the inserting portion 200 through the connecting tube 310. The protective tube 230 is connected both internally and externally, allowing the fluid to fill the gap between the first tube 220 and the sheath 100. Then flows out from the second through hole 212 of the distal tip 210 at the distal end of the inserting portion 200. During suction, the fluid flows from the second through hole 212, then enters the sheath 100 through the insertion portion 200, and is finally discharged through the second outlet 302 via the connecting tube 310 connected to the terminal end of the insertion portion 200. The sheath 100 is quickly connected to the inserting portion 200, which expands the number of channels of the inserting portion 200, increases the volume of the channels, and greatly improves the use performance.

In the second and third embodiments of FIG. 5 and FIG. 6, the connecting structure between the terminal end of the sheath 100 and the holding portion 300 is different. In the second embodiment, the sheath 100 is connected to the holding portion 300 through a hook-like snap-fit structure. In the third embodiment, the sheath 100 is connected to the holding portion 300 through threads. The fluid flow direction in the second embodiment and the third embodiment is consistent with that in the first embodiment. In the fourth embodiment of FIG. 7, the terminal end of the sheath 100 is inserted into the holding portion 300, and the sheath 100 is connected and sealed with the protective tube 230 through the groove and the limiting ring, so that the sheath 100 is fixed at the terminal end of the inserting portion 200. The connecting structure of the sheath 100 and the inserting portion 200 in the above embodiment is different, and the sealing structure is different, but the flow direction of the fluid is consistent with that of the first embodiment.

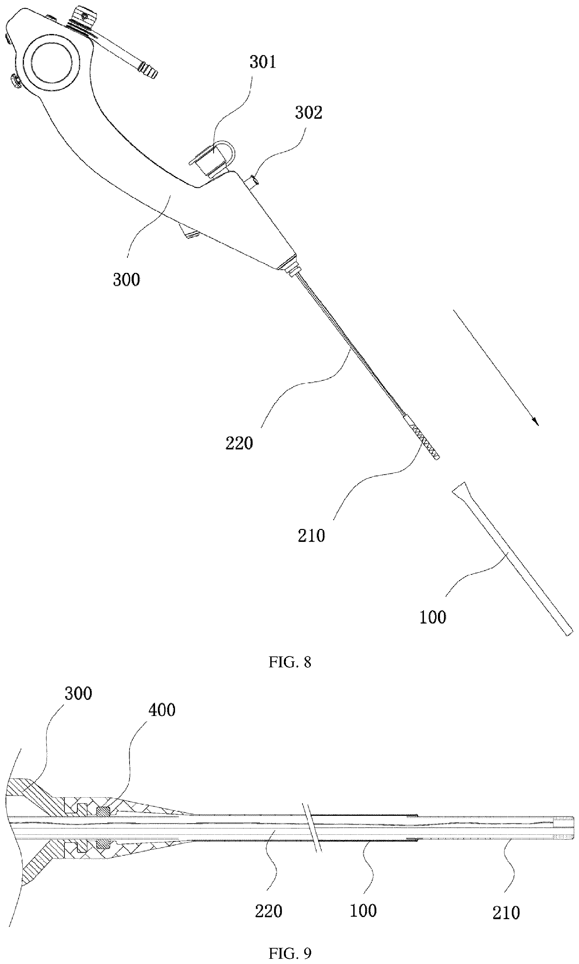

In the fifth embodiment of FIGS. 8 and 9, the inserting portion 200 is not provided with a protective tube 230, the end portion of the sheath 100 is connected to the proximal end of the distal tip 210, and the terminal end of the sheath 100 is connected to the holding portion 300 via a snap-fit structure. The distal end of the connecting tube 310 of the holding portion 300 extends forward into the sheath 100 before extending forward to the connecting structure between the sheath 100 and the holding portion 300. After the sheath 100 is connected, the fluid enters from the second outlet 302 of the holding portion 300 and flows into the inserting portion 200 through the connecting tube 310. The fluid fills the gap between the first tube 220 and the sheath 100. Then flows out from the second through hole 212 of the distal tip 210 at the distal end of the inserting portion 200. During suction, the fluid flows from the second through hole 212, then enters the sheath 100 through the insertion portion 200, and is finally discharged through the second outlet 302 via the connecting tube 310 connected to the terminal end of the insertion portion 200.

In the sixth embodiment of FIG.10, a connecting structure between the sheath 100 and the distal tip 210 is disclosed specifically for the case where the protective tube 230 is not provided. In order to enhance the strength of the distal tip 210, raised reinforcing ribs 600 are arranged inside the terminal end of the distal tip 210, so that the terminal end of the distal tip 210 is less likely to deform and prevent the sheath 100 from being incorrectly connected to the distal tip 210 when it moves forward.

The above is a detailed description of the preferred embodiments of the present disclosure. The described embodiments are only part of the embodiments of the present disclosure, rather than all embodiments. Based on the embodiments of the present disclosure, a person skilled in the art will not violate the present disclosure. Various equivalent modifications or substitutions can be made without departing from the spirit of the present disclosure, and other available embodiments all fall within the protection scope of the present disclosure.

## Claims

1. A medical device with an integrated outer sheath, comprising:
a device body, wherein the device body comprises an inserting portion (200) and a holding portion (300) connected front and back, a first outlet (301) and a second outlet (302) are arranged on the holding portion (300), a tip end of the inserting portion (200) is provided with a distal tip (210), a penetrating first through hole (211) and a second through hole (212) are arranged on the distal tip (210), a first tube (220) is connected to the first through hole (211), and a terminal end of the first tube (220) extends into the holding portion (300) and communicates with the first outlet (301); and
a sheath (100), wherein both ends of the sheath (100) are connected and sealed with the inserting portion (200) respectively through a connecting structure, so that the sheath (100) is detachably and rotatably sleeved outside the inserting portion (200) to form an overtube of the inserting portion (200); a distal end of the sheath (100) is communicated with the second through hole (212) of the distal tip (210), a terminal end of the sheath (100) is communicated with the second outlet (302) of the holding portion (300), the sheath (100) is sleeved outside the first tube (220), and a gap between the sheath (100) and the first tube (220) is a space used by the sheath (100) to transport objects.

2. The medical device with the integrated outer sheath according to claim 1, wherein a connection method of the connecting structure comprises a combination of one or more of the following connection methods: a snap-fit connection, an interference fit connection, a threaded connection, and a magnetic connection.

3. The medical device with the integrated outer sheath according to claim 1, wherein at least one sealing member (400) is arranged between an inner wall of the sheath (100) and an outer wall of the inserting portion (200), the sealing member (400) is positioned on one side of the connecting structure away from an end portion of the inserting portion (200), and the sealing member (400) is fixed on the inner wall of the sheath (100).

4. The medical device with the integrated outer sheath according to claim 1, wherein at least one sealing member (400) is arranged between the inner wall of the sheath (100) and the outer wall of the inserting portion (200), and the sealing member (400) is fixed on the outer wall of the inserting portion (200).

5. The medical device with the integrated outer sheath according to claim 1, wherein an inner diameter of the distal end of the sheath (100) is smaller than an inner diameter of other portions of the sheath (100).

6. The medical device with the integrated outer sheath according to claim 1, wherein the sheath (100) is an elastically deformable flexible tube.

7. The medical device with the integrated outer sheath according to claim 1, wherein both the inner wall and an outer wall of the sheath (100) are provided with a hydrophilic layer.

8. The medical device with the integrated outer sheath according to claim 1, wherein the outer wall of the sheath (100) is provided with raised patterns.

9. The medical device with the integrated outer sheath according to claim 1, wherein the inserting portion (200) further comprises a protective tube (230), at least one end of the protective tube (230) is connected to the distal tip (210) or the holding portion (300), the protective tube (230) is sleeved outside the first tube (220), the sheath (100) is sleeved outside the protective tube (230), and an inside and outside of the protective tube (230) are interconnected.

10. The medical device with the integrated outer sheath according to claim 8, wherein a plurality of leakage holes are arranged on the protective tube (230).

11. The medical device with the integrated outer sheath according to claim 1, wherein an anti-slip structure (130) is arranged on the outer wall of a proximal end of the sheath (100).
